# EUROPÄISCHE PATENTSCHRIFT

(11) **EP 3 383 843 B1**
(45) Veröffentlichungstag und Bekanntmachung des Hinweises auf die Patenterteilung: **25.09.2019**
(21) Anmeldenummer: 16810270.5
(22) Anmeldetag: 28.11.2016
(51) Int. Cl.: C07C 319/14, C07C 323/09, C07C 319/20, C07C 323/65, C07C 323/62, C07C 315/02, C07C 317/44, C07C 317/14

(54) **VERFAHREN ZUR HERSTELLUNG VON 2-ALKYL-4-TRIFLUORMETHYL-3-ALKYLSULFONYLBENZOESÄUREN DURCH CHEMOSELEKTIVE THIOETHEROXIDATION**
METHOD FOR PRODUCING 2-ALKYL-4-TRIFLUOROMETHYL-3-ALKYL SULFONYL BENZOIC ACIDS BY CHEMOSELECTIVE THIOETHER OXIDATION
PROCÉDÉ DE PRÉPARATION D'ACIDES 2-ALKYL-4-TRIFLUOROMÉTHYL-3-ALKYLSULFONYL-BENZOÏQUES PAR OXYDATION CHIMIOSÉLECTIVE DE THIOÉTHER

(30) Priorität: 30.11.2015 EP 15196992
(43) Veröffentlichungstag der Anmeldung: 10.10.2018
(73) Patentinhaber: Bayer CropScience Aktiengesellschaft, 40789 Monheim am Rhein (DE)
(72) Erfinder: SCHOTES, Christoph, 40223 Düsseldorf (DE); SÄMANN, Christoph, 40589 Düsseldorf (DE)
(74) Vertreter: BIP Patents
(86) Internationale Anmeldenummer: PCT/EP2016/078971
(87) Internationale Veröffentlichungsnummer: WO 2017/093172

(56) Entgegenhaltungen:
- WO-A1-2008/125214

## Beschreibung

Die Erfindung betrifft ein Verfahren zur Herstellung von 2-Alkyl-4-trifluormethyl-3-alkylsulfonylbenzoesäuren, die als Intermediate zur Herstellung von agrochemisch wirksamen Stoffen von Nutzen sind.

Aus mehreren Schriften sind agrochemisch wirksame Stoffe bekannt, zu deren Herstellung 2-Alkyl-4-trifluormethyl-3-alkylsulfonylbenzoesäuren benötigt werden. So sind aus WO 2008/125214 A1 herbizid wirksame 4-(4-Trifluormethyl-3-thiobenzoyl)-pyrazole bekannt. Aus WO 2012/126932 A1 sind herbizid wirksame N-(1,3,4-oxadiazol-2-yl)arylcarboxamide, unter anderem auch solche mit einem ähnlichen Suibstitutionsmuster im Phenylring wie die in WO 2008/125214 A1 offenbarten Verbindungen, bekannt.

WO 2008/125214 A1 offenbart auch ein Verfahren zur Herstellung von 2-Methyl-4-trifluormethyl-3-methylsulfonylbenzoesäure. Bei diesem Verfahren wird 3-Fluor-2-methyl-4-trifluormethylbenzoesäure mit Natriumhydrid und Natriumthiomethylat zur 2-Methyl-3-methylthio-4-trifluormethylbenzoesäure umgesetzt, die anschließend zur 2-Methyl-3-methylsulfonyl-4-trifluormethylbenzoesäure oxidiert wird. Nachteile dieses Verfahrens sind die Verwendung der aufwendig herzustellenden 3-Fluor-2-methyl-4-trifluormethyl-benzoesäure sowie die Einführung der Methylgruppe durch Metallierung der 3-Fluor-4-trifluormethyl-benzoesäure mit mindestens 2 Moläquivalenten Butyllithium bei tiefer Temperatur,gefolgt von einer Umsetzung mit dem toxischen Methyliodid. Dieses Verfahren ist aufwendig und wegen der nur geringen Ausbeute bei der Einführung der Methylgruppe (50,7% der Theorie) zudem unökonomisch.

Ebenfalls bekannt sind Verfahren zur Herstellung von substituierten Benzoesäuren durch übergangsmetallkatalysierte Cyanierungen von Chloraromaten und anschließender Verseifung der Cyanogruppe zur Säuregruppe. Nachteilig hierbei ist, dass hochgiftige Cyanide sowie teure und chemisch empfindliche Katalysatoren zum Einsatz kommen. Auch muss hier eine aufwendige Bearbeitung der Abfallströme erfolgen, um Schäden für Mensch und Umwelt auszuschließen. Weiterhin ist bekannt, dass man Sulfoxidgruppen mit Hilfe metallorganischer Verbindungen, wie Lithiumalkyl oder Lithiumaryle gegen Metall austauschen, und die entstandene Spezies anschließend mit Kohlendioxid zu einer Carbonsäure umsetzen kann. Jedoch treten bei diesem Reaktionstyp je nach Art der Substituenten unerwünschte Reaktionen auf.

Aufgabe der vorliegenden Erfindung ist die Bereitstellung eines Verfahrens zur Herstellung von 2-Alkyl-4-trifluormethyl-3-alkylsulfonylbenzoesäuren, welches die Nachteile der aus dem Stand der Technik bekannten Verfahren überwindet.

Es wurde nun gefunden, dass 2-Alkyl-4-trifluormethyl-3-alkylsulfonylbenzoesäuren, ausgehend von 1,3-Dichlor-2-alkyl-4-trifluormethylbenzolen, ohne Einsatz von Cyaniden oder Übergangsmetallkatalysatoren hergestellt werden können durch die Reaktionsfolge einer doppelten Thiolierung, selektiver Oxidation einer Thioethergruppe, Austausch der Sulfoxidgruppe gegen ein Metall, Carboxylierung und Oxidation der verbleibenden Thioethergruppe.

Ein Gegenstand vorliegender Erfindung ist somit ein Verfahren zur Herstellung von 2-Alkyl-4-trifluormethyl-3-alkylsulfonylbenzoesäuren der allgemeinen Formel (I), dadurch gekennzeichnet, dass
a) in einem ersten Schritt ein 1,3-Dichlor-2-alkyl-4-trifluormethylbenzol (II) mit einem Thiolat (IV) zu einem Aryl-bisthioether (III) umgesetzt wird,
b) in einem zweiten Schritt der Aryl-bisthioether (III) mit einem Oxidationsmittel selektiv zu einem Aryl-monosulfoxid-monothioether (V) umgesetzt wird,
c) in einem dritten Schritt ein Austausch der Sulfoxidgruppe gegen ein Metall stattfindet und die so erhaltene metallorganische Verbindung zur Benzoesäure (VII) umgesetzt wird, und
d) in einem vierten Schritt die verbleibende Thiogruppe mit einem Oxidationsmittel, gegebenenfalls in Gegenwart eines Oxidationskatalysators, oxidiert wird: 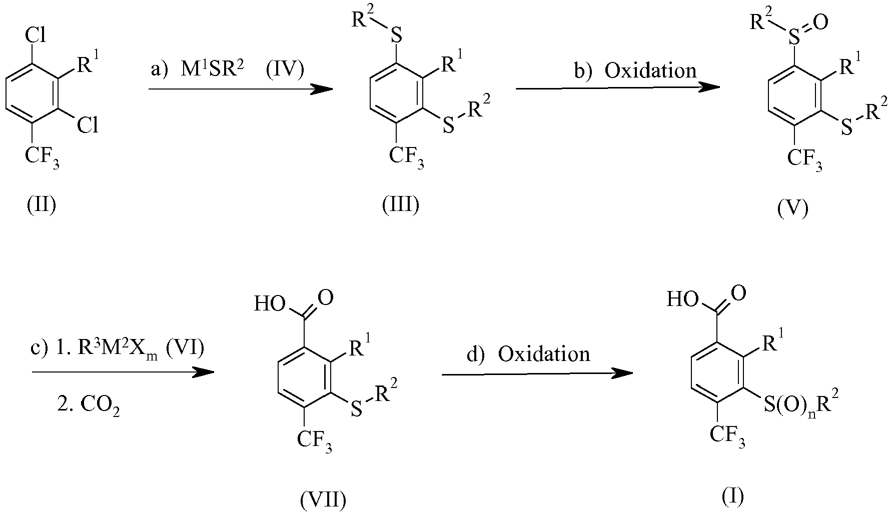 und
e) worin die Substituenten wie nachfolgend definiert sind:
   - R¹ und R²: bedeuten unabhängig voneinander C₁-C₄-Alkyl oder durch s Reste aus der Gruppe bestehend aus Chlor, Fluor, Methoxy und Ethoxy substituiertes Phenyl,
   - R³: bedeutet C₁-C₁₀ Alkyl oder durch s Reste aus der Gruppe bestehend aus Chlor, Fluor, Methoxy und Ethoxy substituiertes Phenyl,
   - M¹: bedeutet Lithium, Natrium oder Kalium,
   - M²: bedeutet Magnesium, Lithium oder Zink,
   - X: bedeutet Chlorid, Bromid oder Iodid,
   - m: bedeutet 0 oder 1,
   - n: bedeutet 1 oder 2,
   - s: bedeutet 0, 1, 2 oder 3.

Wesentliche Vorteile des erfindungsgemäßen Verfahrens sind:
- die gute Differentierung der beiden Chlorsubstituenten an Verbindung (II) ohne Verwendung eines Übergangsmetallkatalysators,
- die selektive Thioetheroxidation mit günstigen Reagenzien wie Peressigsäure oder H₂O₂,
- die Generierung der Carbonsäuregruppe mit Hilfe eines einfachen organometallischem Reagenzes, in Verbindung mit günstigem und unkompliziert handhabbaren Kohlenstoffdioxid.

In den Formeln (I), (II), (III), (IV), (V), (VI) und (VII) können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl.

Als Thiolat (IV) sind Natriumthiomethylat (NaSMe) und Kaliumthiomethylat (KSMe) besonders gut geeignet.

Im ersten Schritt des erfindungsgemäßen Verfahrens wird die Verbindung der allgemeinen Formel (IV) in einem Mengenverhältnis von 2:1 bis 4:1 Moläquivalenten bezogen auf die Verbindung der allgemeinen Formel (II) eingesetzt. Bevorzugt ist ein Mengenverhältnis von 2:1 bis 3:1, besonders bevorzugt 2,5:1.

Die Verbindungen der allgemeinen Formel (IV) können sowohl in-situ also auch ex-situ aus den entsprechenden Thiolen und einer Base, wie Alkalimetallhydroxide, Erdalkalimetallhydroxide, Alkalimetallkarbonate, Alkalimetallacetate, Alkalimetallalkoholate und organischen Basen, hergestellt werden. Geeignete Basen sind LiOH, NaOH, KOH, Ca(OH)₂, Na₂CO₃, K₂CO₃, Cs₂CO₃, NaOAc, KOAc, LiOAc, NaOMe, NaOEt, NaO-t-Bu, KO-t-Bu, Trialkylamine, Alkylpyridine, Phosphazene und 1,8-Diazabicyclo[5.4.0]undecen.

Die Reaktion des ersten Schritts des erfindungsgemäßen Verfahrens wird in der Regel in einem Lösungsmittel wie Acetonitril, Dimethylformatid, Dimethylacetamid, Dimethylsulfoxid oder N-Methyl-2-pyrrolidon durchgeführt. Bevorzugt sind Dimethylsulfoxid, Dimethylacetamid, Dimethylformamid und N-Methyl-2-pyrrolidon, besonders bevorzugt Dimethylsulfoxid.

Die Reaktion im ersten Schritt des erfindungsgemäßen Verfahrens wird in der Regel bei einer Temperatur von 0 bis 100° C, vorzugsweise 20 bis 70° C, besonders bevorzugt 30 bis 40° C, durchgeführt. Die Reaktion kann auch unter erhöhtem oder veringertem Druck durchgeführt werden.

Durch Überschüsse der Verbindung (IV) kann es zu einer teilweisen Demethylierung der Verbindung (III) kommen. Dies kann kompensiert werden, indem Alkylierungsmittel wie Me₂SO₄, MeI, MeBr, MeCl oder Dimethylcarbonat nachgesetzt werden. Bevorzugt ist Me₂SO₄.

Im zweiten Schritt des erfindungsgemäßen Verfahrens wird das Oxidationsmittel im Mengenverhältnis von 2:1 bis 4:1 Moläquivalenten bezogen auf die Verbindung der allgemeinen Formel (III) eingesetzt. Bevorzugt sind 1,5:1 bis 3,5:1, besonders bevorzugt 3:1. Geeignete Oxidationsmittel sind H₂O₂, Peressigsäure oder meta-Chlorperbenzoesäure. Bevorzugt ist H₂O₂. Überraschenderweise wird dabei selektiv die zur Trifluomethylgruppe para-ständige Thioethergruppe zum Sulfoxid oxidiert.

Die Reaktion des zweiten Schritts des erfindungsgemäßen Verfahrens wird in der Regel in einem Lösungsmittel wie Acetonitril, Essigeseter, Butylester, Toluol, Chlorbenzol, Dichlormethan, Essigsäure oder Propionsäure durchgeführt. Bevorzugt sind Dichlormethan, Essigsäure und Propionsäure, besonders bevorzugt Essigsäure und Propionsäure.

Es können bei der Reaktion im zweiten Schritt des erfindungsgemäßen Verfahrens auch dem Fachmann bekannte Oxidationskatalysatoren auf Basis von Vanadium oder Eisen eingesetzt werden.

Die Reaktion im zweiten Schritt des erfindungsgemäßen Verfahrens wird in der Regel bei einer Temperatur von 0 bis 50° C, bevorzugt bei 10 bis 30° C und besonders bevorzugt bei 20 °C durchgeführt. Die Reaktion kann auch unter erhöhtem oder veringertem Druck durchgeführt werden.

Im dritten Schritt des erfindungsgemäßen Verfahrens wird die Sulfoxidgruppe in Verbindungen vom Typ (V) mit Hilfe einer metallorganischen Verbindung gegen ein Metall ausgetauscht. Als metallorganische Reagentien kommen Verbindungen der allgemeinen Formel (VI) zum Einsatz, bei denen M² Magnesium, Zink oder Lithium ist und R³ ein Rest aus der Gruppe bestehend aus Methyl, Ethyl, iso-Propyl, Butyl, iso-Butyl, tert-Butyl, Hexyl, Phenyl, Vinyl, Allyl und Mesityl ist. Bevorzugt sind Grignard Verbindungen, worin M² = Mg, X = Cl oder Br und R³ ein Rest aus der Gruppe bestehend aus iso-Propyl, Butyl, Hexyl oder Ethyl ist. Besonders bevorzugt sind iso-Propylmagnesiumchlorid, Ethylmagnesiumbromid und Butylmagnesiumchlorid.

Die metallorganischen Reagentien können auch in Kombination mit LiCl eingesetzt werden. Die Reagentien werden als Lösungen eingesetzt in Lösungsmitteln wie Tetrahydrofuran, 2-Methyltetrahydrofuran, Dioxan, Diethylether, tert-Butylmethylether, oder Cyclopentylmethylether. Auch Mischungen dieser Lösungsmittel sind geeignet. Die metallorganischen Reagentien werden in einem Mengenverhältnis von 2:1 bis 0,7:1 Moläquivalenten bezogen auf die Verbindung der allgemeinen Formel (V) eingesetzt. Bevorzugt sind 1:1 bis 1,5:1, besonders bevorzugt 1,1:1.

Die Verbindungen der allgemeinen Formel (V) werden bei der Reaktion im dritten Schritt des erfindungsgemäßen Verfahrens in der Regel in einem Lösungsmittel gelöst. Geeignete Lösungsmittel sind Tetrahydrofuran, 2-Methyltetrahydrofuran, Dioxan, Diethylether, tert-Butylmethylether, Toluol, Xylol und Cyclopentylmethylether. Bevorzugt sind Toluol, Tetrahydrofuran und 2-Methyltetrahydrofuran, besonders bevorzugt ist Toluol.

Nach Bildung der metallorganischen Spezies wird diese mit einem Elektrophil wie Kohlenstoffdioxid, Dimethylcarbonat oder Diethylcarbonat abgefangen. Bevorzugt ist Kohlenstoffdioxid. Die Elektrophile werden in der Regel im Überschuss eingesetzt (1,1 - 20 Moläquivalente).

Die Reaktion im dritten Schritt des erfindungsgemäßen Verfahrens wird in der Regel bei einer Temperatur von -80 bis 0° C, vorzugsweise -30 bis -10° C, besonders bevorzugt bei -25 °C durchgeführt. Die Reaktion kann auch unter erhöhtem oder veringertem Druck durchgeführt werden.

Im vierten Schritt des erfindungsgemäßen Verfahrens wird die Thiogruppe der Verbindung (VII) mit Wasserstoffperoxid oxidiert, gegebenenfalls in Gegenwart eines Oxidationskatalysators. Geeignete Oxydationskatalysatoren sind Na₂WO₄, Na₂MoO₄ sowie deren Hydrate, sowie Schwefelsäure in Kombination mit einer organischen Säure, wie Essigsäure, Ameisensäure oder Trifluoressigsäure.

Die Oxidationskatalysatoren werden in Mengen von 1 bis 20 Molprozent, bezogen auf die Verbindung der allgemeinen Formel (VII) eingesetzt. Bevorzugt sind 5 bis 15 Molprozent, besonders bevorzugt 10 Molprozent.

Wasserstoffperoxid wird in einer Menge von 2 bis 10, bevorzugt 3 bis 8, besonders bevorzugt 3,5 bis 5 Moläquivalenten, bezogen auf die Verbindung der allgemeinen Formel (VII), eingesetzt. Üblicherweise wird das Wasserstoffperoxid als 20-35 %-ige wässrige Lösung eingesetzt.

Die Reaktion im vierten Schritt des erfindungsgemäßen Verfahrens wird in der Regel bei einer Temperatur von 30 bis 110° C, vorzugsweise 40 bis 80° C, besonders bevorzugt 50 bis 70° C, durchgeführt. Die Reaktion kann auch unter erhöhtem oder veringertem Druck durchgeführt werden.

Die Reaktion des vierten Schritts des erfindungsgemäßen Verfahrens wird in der Regel in einem Lösungsmittel durchgeführt. Geeignete Lösungsmittel sind Toluol, Chlorbenzol, Dichlorbenzol, Essigsäureethylester, Essigsäurebutylester, Essigsäure, Ameisensäure und Wasser. Bevorzugt sind Toluol, Essigsäureethylester, Essigsäurebutylester, Ameisensäure und Wasser. Besonders bevorzugt sind Toluol, Essigsäurebutylester, Essigsäure und Wasser.

Verbindungen der Formel (III), worin R¹ und R² für bestimmte Substituenten stehen, sind neu und eignen sich sehr gut als Ausgangsmaterial für den zweiten Schritt des erfindungsgemäßen Verfahrens. Ein weiterer Gegenstand vorliegender Erfindung sind somit Verbindungen der Formel (III), 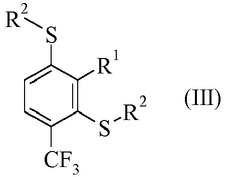 worin
- R¹ und R²: bedeuten unabhängig voneinander C₁-C₄-Alkyl oder durch s Reste aus der Gruppe bestehend aus Chlor, Fluor, Methoxy und Ethoxy substituiertes Phenyl,
- s: bedeutet 1, 2 oder 3.

Bevorzugt bedeuten R¹ und R² in Formel (III) unabhängig voneinander Methyl, Ethyl, n-Propyl, iso-Propyl oder n-Butyl. Besonders bevorzugt bedeuten R¹ und R² jeweils Methyl.

Verbindungen der Formel (V) sind ebenfalls neu und eignen sich sehr gut als Ausgangsmaterial für den dritten Schritt des erfindungsgemäßen Verfahrens. Ein weiterer Gegenstand vorliegender Erfindung sind somit Verbindungen der Formel (V), 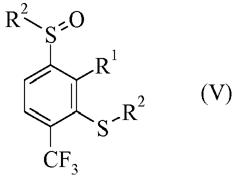 worin
- R¹ und R²: bedeuten unabhängig voneinander C₁-C₄-Alkyl oder durch s Reste aus der Gruppe bestehend aus Chlor, Fluor, Methoxy und Ethoxy substituiertes Phenyl,
- s: bedeutet 1, 2 oder 3.

Bevorzugt bedeuten R¹ und R² in Formel (V) unabhängig voneinander Methyl, Ethyl, n-Propyl, iso-Propyl oder n-Butyl. Besonders bevorzugt bedeuten R¹ und R² jeweils Methyl.

Werden in Schritt zwei des erfindungsgemäßen Verfahrens hohe Überschüsse des Oxidationsmittels, sowie höhere Temperaturen und längere Reaktionszeiten eingesetzt, erhält man Verbindungen der allgemeinen Formel (VIII). Diese sind ebenfalls neu und können als Ausgangsmaterialien anderer agrochemisch wirksamer Verbindungen dienen. Ein weiterer Gegenstand vorliegender Erfindung sind somit Verbindungen der allgemeinen Formel (VIII), 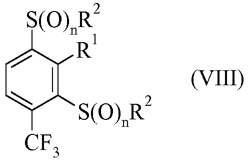 worin
- R¹ und R²: bedeuten unabhängig voneinander C₁-C₄-Alkyl oder durch s Reste aus der Gruppe bestehend aus Chlor, Fluor, Methoxy und Ethoxy substituiertes Phenyl,
- s: bedeutet 1, 2 oder 3
- n: bedeutet 1 oder 2.

Die nachfolgenden Beispiele erläutern die Erfindung näher ohne sie darauf einzuschränken.

### Herstellung von 2-Methyl-3-(methylsulfonyl)-4-(trifluormethyl)benzoesäure

### Schritt 1: 2-Methyl-1,3-bis(methylsulfanyl)-4-(trifluoromethyl)benzol

2,4-Dichlor-3-methyltrifluorbenzol (29,7 g, 130 mmol, 1 equiv) werden in 150 mL DMSO vorgelegt und 10 min gerührt, bis eine klare Lösung entsteht. NaSMe (24 g, 325 mmol, 2,5 equiv) werden unter Eiskühlung in Portionen zugegeben. Nach Abklingen der Exothermie wird auf 40 °C erwärmt und für 20 h nachgerührt. Nach Abkühlen auf Raumtemperatur werden 3,3 g (26 mmol, 0,2 equiv) Me₂SO₄ zugesetzt und 30 min nachgerührt. Unter Kühlung werden 30 mL 20%ige NaOH zugesetzt und 30 min nachgerührt. Es wird mit 250 mL Wasser verdünnt und 3 mal mit Methylcyclohexan extrahiert. Die vereinten organischen Phasen werden mit Wasser und gesättigter Natriumchloridlösung gewaschen, über Na₂SO₄ getrocknet und das Lösungsmittel wird unter reduziertem Druck entfernt. Es werden 29,5 g von 2-Methyl-1,3-bis(methylsulfanyl)-4-(trifluoromethyl)benzol (88% Ausbeute) als farbloses Öl erhalten.
¹H NMR (600MHz, CDCl₃) δ = 7,53 (d, *J* = 8,5 Hz, 1H), 7,14 (d, *J* = 8,5 Hz, 1H), 2,65 (s, 3H), 2,50 (s, 3H), 2,26 (s, 3H). GC/MS: m/e = 252 (M); 237 (M-H₃C^{.}).

### Schritt 2: 2-Methyl-3 -methylsulfanyl-1 -methylsulfinyl-4-(trifluoromethyl)benzol

2-Methyl-1,3-bis(methylsulfanyl)-4-(trifluoromethyl)benzol (2 g, 7,9 mmol, 1 equiv) wird mit 0,5 mL Essigsäure (8,7 mmol, 1,1 equiv) vorgelegt. 2,05 mL einer 35%igen H₂O₂ Lösung (23,8 mmol, 3 equiv) werden über eine Stunde eindosiert und es wird 2 h nachgerührt bei Raumtemperatur. Der Ansatz wird mit einer Bisulfitlösung versetzt um Peroxide zu vernichten. Die Essigsäure wird am Rotationsverdampfer entfernt. Es werden Wasser und Dichlormethan zugeben und die organische Phase abgetrennt. Diese wird mit Wasser und Bicarbonatlösung gewaschen, dann wird das Lösungsmitel am Rotationsverdampfer entfernt. Man erhält 2-Methyl-3-methylsulfanyl-1-methylsulfinyl-4-(trifluoromethyl)benzol als weißen Feststoff in einer Ausbeute von 75%. Gegebenenfalls enthaltene Mengen an Bis-sulfoxid (allgemeine Verbindung VIII mit R¹=R²=Me, n=1) können durch Umkristallisation aus MeOH/Wasser oder chromatographisch abgetrennt werden.
¹H-NMR (600 MHz, CDCl₃): δ 8,10 (d, *J* = 8,3 Hz, 1H) 7,85 (d, *J* = 8.3 Hz, 1H), 2,71 (s, 3H), 2,64 (s, 3H), 2,29 (s, 3H). GC/MS: m/e = 268.

### Schritt 3: 2-Methyl-3-methylsulfanyl-4-(trifluoromethyl)benzoesäure

Sämtliche Operationen werden unter Schutzgas ausgeführt. Es werden 60 mL Toluol vorgelegt und auf -25 °C abgekühlt. 2-Methyl-3-methylsulfanyl-1-methylsulfinyl-4-(trifluoromethyl)benzol (10 g, 36,4 mmol, 1 equiv) wird in 40 mL Toluol gelöst. In einem weiteren Gefäß werden 15 mL einer 3.26 molaren EtMgBr/2-Methyltetrahydrofuran Lösung vorgelegt und mit 24,3 mL Tetrahydrofuran und 9,3 mL 2-Methyltetrahydrofuran verdünnt. Der Titer der resultierenden Grignard Lösung beträgt 1,03 M.

Nun werden die Lösungen von 2-Methyl-3-methylsulfanyl-1-methylsulfinyl-4-(trifluoromethyl)benzol und EtMgBr parallel in den Reaktionskolben bei -25 °C über 30 min eingetropft. Es wird 40 min bei dieser Temperatur nachgerührt, dann wird trockenes CO₂ eingeleitet. Die Innentemperatur wird unter - 10 °C gehalten. Die Reaktionslösung lässt man auf Raumtemperatur kommen, und versetzt sie dann mit konzentrierter HCl. Die Phasen werden getrennt und die wässrige Phase 2 mal mit Toluol nachgewaschen. Die vereinten organischen Phasen werden mit IN wässriger NaOH basisch gestellt. Die wässrige Phase wird abgetrennt und die organische Phase 2 mal mit IN NaOH nachextrahiert. Die wässrige Phase wird mit konzentrierter HCl auf pH 0-1 gestellt und 3 mal mit Essigester extrahiert. Die vereinten organischen Phasen werden über Na₂SO₄ getrocknet, anschließend werden die Lösungsmittel unter reduziertem Druck entfernt. Es werden 6,2 g 2-Methyl-3-methylsulfanyl-4-(trifluoromethyl) benzoesäure (59% Aubeute nach NMR Quantifizierung) als leicht gelblicher Feststoff isoliert.

### Schritt 4: 2-Methyl-3 -(methylsulfonyl)-4-(trifluormethyl)benzoesäure

2-Methyl-3-(methylsulfanyl)-4-(trifluormethyl)benzoesäure (9,6 g, 38 mmol, 1 equiv) werden in 60 mL Essigsäure-n-butylester gelöst und 1,1 g (3,8 mmol, 0.1 equiv) Natriumwolframat-dihydrat zugegeben. Der Ansatz wird intensiv gerührt und auf 55° C erhitzt. Per Spritzenpumpe werden in 2 Stunden 16,2 ml (190 mmol, 5 equiv) 35%ige Wasserstoffperoxidlösung bei 55-60° Innentemperatur zudosiert. Das Gemisch wird 8 bis 10 Stunden bei dieser Temperatur weitergerührt. Danach wird der Ansatz abgekühlt und mit verd. HCl auf pH = 0 gestellt. Die Reaktionslösung wird auf 60 °C erwärmt, die Phasen werden warm getrennt. Der Großteil des Essigsäure-n-butylesters wird unter reduziertem Druck entfernt. Der entstehende dicke Brei wird abgekühlt und mit wenig Toluol versetzt. Der Niederschlag wird abgesaugt, mit Wasser gewaschen und getrocknet. Man erhält 8,7 g 2-Methyl-3-(methylsulfonyl)-4-(trifluormethyl)benzoesäure (81% Ausbeute) als weißer Feststoff.

### Herstellung der allgemeinen Verbindungen VIII, Beispiel: 2-Methyl-1,3-bis(methylsulfonyl)-4-(trifluoromethyl)benzol

Es werden 0,7 g 2-Methyl-1,3-bis(methylsulfanyl)-4-(trifluoromethyl)benzol (2,77 mmol, 1 equiv) in Dichlormethan (50 mL) vorgelegt. 5 g meta-Chlorperbenzoesäure (22 mmol, 8 equiv) werden bei Raumtemperatur zugesetzt und es wird für 20 h gerührt. Die enstandene Suspension wird filtriert, das Filtrat mit wässriger Bicarbonat und wässriger Natriumchloridlösung gewaschen. Nach Entfernen des Lösungmittels unter reduziertem Druck, erhält man einen weißen Feststoff, den man Zwecks Umkristallisation in 50 mL Isopropanol erhitzt, anschliessend abkühlt und filtiert. Nach Trocknung erhält man 0,76 g 2-Methyl-1,3-bis(methylsulfonyl)-4-(trifluoromethyl)benzol (86% Ausbeute) als weiße Kristalle.
¹H-NMR (600 MHz, CD₃CN): δ 8,48 (d, 1H, *J* = 8,6 Hz) 8,08 (d, 1H, *J* = 8,6 Hz), 3,35 (s, 3H), 3,26 (s, 3H), 3,11 (s, 3H). LC/MS (ESI-neg): m/e = 315,0 (M-1).

## Patentansprüche

1. Verfahren zur Herstellung von 2-Alkyl-4-trifluomethyl-3-alkylsulfonylbenzoesäuren der allgemeinen Formel (I), **dadurch gekennzeichnet, dass**
a) in einem ersten Schritt ein 1,3-Dichlor-2-alkyl-4-trifluormethylbenzol (II) mit einem Thiolat (IV) zu einem Aryl-bisthioether (III) umgesetzt wird,
b) in einem zweiten Schritt der Aryl-bisthioether (III) mit einem Oxidationsmittel selektiv zu einem Aryl-monosulfoxid-monothioether (V) umgesetzt wird,
c) in einem dritten Schritt ein Austausch der Sulfoxidgruppe gegen ein Metall stattfindet und die so erhaltene metallorganische Verbindung zur Benzoesäure (VII) umgesetzt wird, und
d) in einem vierten Schritt die verbleibende Thiogruppe mit einem Oxidationsmittel, gegebenenfalls in Gegenwart eines Oxidationskatalysators, oxidiert wird: 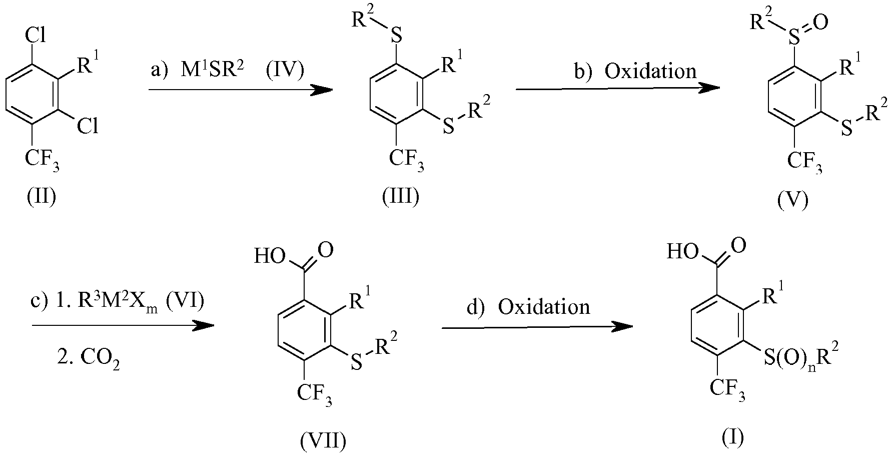 und
e) worin die Substituenten wie nachfolgend definiert sind:
R¹ und R² bedeuten unabhängig voneinander C₁-C₄-Alkyl oder durch s Reste aus der Gruppe bestehend aus Chlor, Fluor, Methoxy und Ethoxy substituiertes Phenyl,
R³ bedeutet C₁-C₁₀ Alkyl oder durch s Reste aus der Gruppe bestehend aus Chlor, Fluor, Methoxy und Ethoxy substituiertes Phenyl,
M¹ bedeutet Lithium, Natrium oder Kalium,
M² bedeutet Magnesium, Lithium oder Zink,
X bedeutet Chlorid, Bromid oder Iodid,
m bedeutet 0 oder 1,
n bedeutet 1 oder 2,
s bedeutet 0, 1, 2 oder 3.

2. Verfahren nach Anspruch 1, worin als Thiolat (IV) NaSMe oder KSMe verwendet wird.

3. Verfahren nach Anspruch 1 oder 2, worin das Thiolat (IV) in einem Molverhältnis von 2:1 bis 3:1, bezogen auf die Verbindung der allgemeinen Formel (II) eingesetzt wird.

4. Verfahren nach einem der Ansprüche 1 bis 3, worin im ersten Schritt Dimethylsulfoxid, Dimethylacetamid, Dimethylformamid oder N-Methyl-2-pyrrolidon als Lösungsmittel verwendet werden.

5. Verfahren nach einem der Ansprüche 1 bis 4, worin im zweiten Schritt H₂O₂ zur selektiven Oxidation verwendet wird.

6. Verfahren nach einem der Ansprüche 1 bis 5, worin im zweiten Schritt Essigsäure, Propionsäure oder Dichlormethan als Lösungsmittel eingesetzt werden.

7. Verfahren nach einem der Ansprüche 1 bis 6, worin als Oxidationskatalysator Na₂WO₄ in einer Menge von 5 bis 15 Molprozent, und Wasserstoffperoxid in einer Menge von 3 bis 8 Moläquivalenten, jeweils bezogen auf die Verbindung der allgemeinen Formel (VII), eingesetzt werden.

8. Verfahren nach einem der Ansprüche 1 bis 7, worin im dritten Schritt Ethylmagnesiumbromid, Butylmagnesiumchlorid oder iso-Propylmagnesiumchlorid als organometallische Verbindungen eingesetzt werden.

9. Verfahren nach einem der Ansprüche 1 bis 8, worin im dritten Schritt Toluol, Tetrahydrofuran oder 2-Methyltetrahydrofuran als Lösungsmittel eingesetzt werden.

10. Verbindungen der Formel (III), 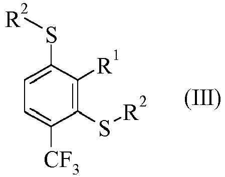 worin
R¹ und R² bedeuten unabhängig voneinander C₁-C₄-Alkyl oder durch s Reste aus der Gruppe bestehend aus Chlor, Fluor, Methoxy und Ethoxy substituiertes Phenyl,
s bedeutet 1, 2 oder 3.

11. Verbindungen nach Anspruch 10, worin R¹ und R² Methyl, Ethyl n-Propyl oder iso-Propyl oder n-Butyl bedeuten.

12. Verbindungen der Formel (V), 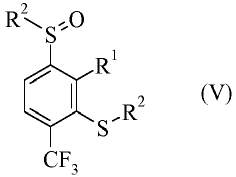 worin
R¹ und R² bedeuten unabhängig voneinander C₁-C₄-Alkyl oder durch s Reste aus der Gruppe bestehend aus Chlor, Fluor, Methoxy und Ethoxy substituiertes Phenyl,
s bedeutet 1, 2 oder 3.

13. Verbindungen nach Anspruch 12, worin R¹ und R² unabhängig voneinander Methyl, Ethyl, n-Propyl, iso-Propyl oder n-Butyl bedeuten.

14. Verbindungen der Formel (VIII), 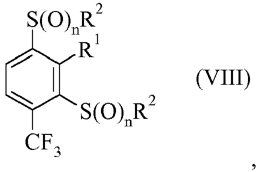 worin
R¹ und R² bedeuten unabhängig voneinander C₁-C₄-Alkyl oder durch s Reste aus der Gruppe bestehend aus Chlor, Fluor, Methoxy und Ethoxy substituiertes Phenyl,
s bedeutet 1, 2 oder 3,
n bedeutet 1 oder 2.

15. Verbindungen nach Anspruch 14, worin R¹ und R² unabhängig voneinander Methyl, Ethyl, n-Propyl, iso-Propyl oder n-Butyl bedeuten.

## Claims

1. Process for the preparation of 2-alkyl-4-trifluoromethyl-3-alkylsulfonylbenzoic acids of the general formula (I), **characterized in that**
a) in a first step a 1,3-dichloro-2-alkyl-4-trifluoromethylbenzene (II) is reacted with a thiolate (IV) to give an aryl bisthioether (III),
b) in a second step the aryl bisthioether (III) is reacted with an oxidizing agent selectively to give an aryl monosulfoxide monothioether (V),
c) in a third step an exchange of the sulfoxide group for a metal takes place and the organometallic compound thus obtained is converted to the benzoic acid (VII), and
d) in a fourth step the remaining thio group is oxidized with an oxidizing agent, optionally in the presence of an oxidation catalyst: 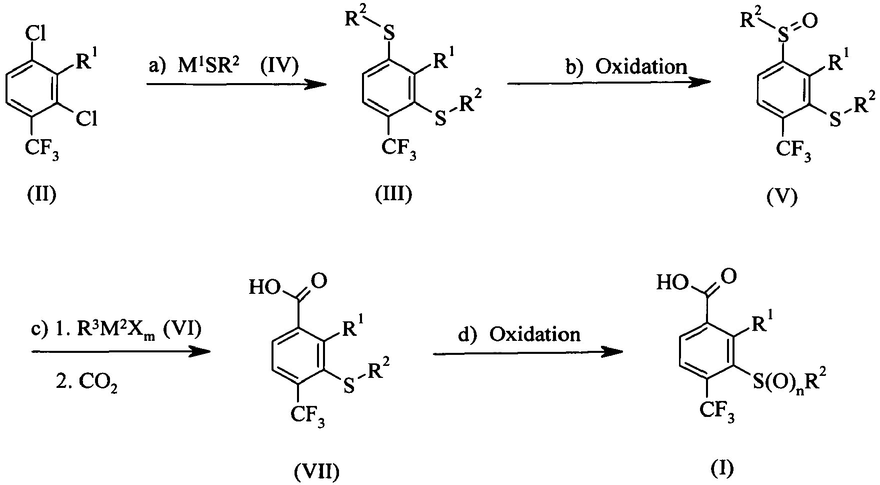 and
e) in which the substituents are defined as follows:
R¹ and R² are, independently of one another, C₁-C₄-alkyl or phenyl substituted by s radicals from the group consisting of chlorine, fluorine, methoxy and ethoxy,
R³ is C₁-C₁₀ alkyl or phenyl substituted by s radicals from the group consisting of chlorine, fluorine, methoxy and ethoxy,
M¹ is lithium, sodium or potassium,
M² is magnesium, lithium or zinc,
X is chloride, bromide or iodide,
m is 0 or 1,
n is 1 or 2,
s is 0, 1, 2 or 3.

2. Process according to Claim 1, in which NaSMe or KSMe is used as thiolate (IV).

3. Process according to Claim 1 or 2, in which the thiolate (IV) is used in a molar ratio of 2:1 to 3:1, based on the compound of the general formula (II).

4. Process according to one of Claims 1 to 3, in which, in the first step, dimethyl sulfoxide, dimethylacetamide, dimethylformamide or N-methyl-2-pyrrolidone are used as solvents.

5. Process according to one of Claims 1 to 4, in which, in the second step, H₂O₂ is used for the selective oxidation.

6. Process according to one of Claims 1 to 5, in which, in the second step, acetic acid, propionic acid or dichloromethane are used as solvents.

7. Process according to one of Claims 1 to 6, in which Na₂WO₄ in an amount of 5 to 15 mol%, and hydrogen peroxide in an amount of 3 to 8 mol equivalents, in each case based on the compound of the general formula (VII), are used as oxidation catalyst.

8. Process according to one of Claims 1 to 7, in which, in the third step, ethylmagnesium bromide, butylmagnesium chloride or isopropylmagnesium chloride are used as organometallic compounds.

9. Process according to one of Claims 1 to 8, in which, in the third step, toluene, tetrahydrofuran or 2-methyltetrahydrofuran are used as solvent.

10. Compounds of the formula (III), 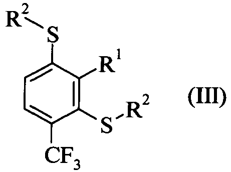 in which
R¹ and R² are, independently of one another, C₁-C₄-alkyl or phenyl substituted by s radicals from the group consisting of chlorine, fluorine, methoxy and ethoxy,
s is 1, 2 or 3.

11. Compounds according to Claim 10, in which R¹ and R² are methyl, ethyl n-propyl or isopropyl or n-butyl.

12. Compounds of the formula (V), 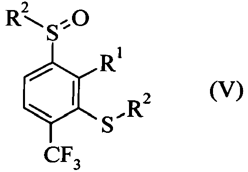 in which
R¹ and R² are, independently of one another, C₁-C₄-alkyl or phenyl substituted by s radicals
from the group consisting of chlorine, fluorine, methoxy and ethoxy,
s is 1, 2 or 3.

13. Compounds according to Claim 12, in which R¹ and R², independently of one another, are methyl, ethyl, n-propyl, isopropyl or n-butyl.

14. Compounds of the formula (VIII), 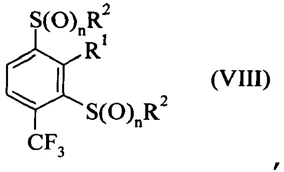 in which
R¹ and R² are, independently of one another, C₁-C₄-alkyl or phenyl substituted by s radicals from the group consisting of chlorine, fluorine, methoxy and ethoxy,
s is 1, 2 or 3,
n is 1 or 2.

15. Compounds according to Claim 14, in which R¹ and R², independently of one another, are methyl, ethyl, n-propyl, isopropyl or n-butyl.

## Revendications

1. Procédé de fabrication d'acides 2-alkyl-4-trifluorométhyl-3-alkylsulfonylbenzoïques de la formule générale (I), **caractérisé en ce que**
a) lors d'une première étape, un 1,3-dichloro-2-alkyl-4-trifluorométhylbenzènxe (II) est mis en réaction avec un thiolate (IV) pour former un aryl-bisthioéther (III),
b) lors d'une deuxième étape, l'aryl-bisthioéther (III) est mis en réaction sélectivement avec un oxydant pour former un aryl-monosulfoxyde-monothioéther (V),
c) lors d'une troisième étape, un remplacement du groupe sulfoxyde par un métal a lieu, et le composé métallo-organique ainsi obtenu est transformé en l'acide benzoïque (VII), et
d) lors d'une quatrième étape, le groupe thio restant est oxydé avec un oxydant, éventuellement en présence d'un catalyseur d'oxydation : 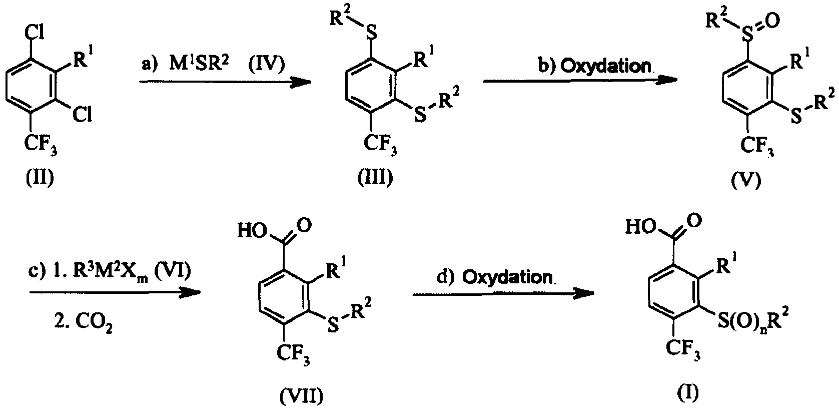 et
e) les substituants étant tels que définis ci-après :
R¹ et R² signifient indépendamment l'un de l'autre alkyle en C₁-C₄, ou phényle substitué par s radicaux du groupe constitué par chlore, fluor, méthoxy et éthoxy,
R³ signifie alkyle en C₁-C₁₀, ou phényle substitué par s radicaux du groupe constitué par chlore, fluor, méthoxy et éthoxy,
M¹ signifie lithium, sodium ou potassium,
M² signifie magnésium, lithium ou zinc,
X signifie chlorure, bromure ou iodure,
m signifie 0 ou 1,
n signifie 1 ou 2,
s signifie 0, 1, 2 ou 3.

2. Procédé selon la revendication 1, dans lequel NaSMe ou KSMe est utilisé en tant que thiolate (IV).

3. Procédé selon la revendication 1 ou 2, dans lequel le thiolate (IV) est utilisé en un rapport molaire de 2:1 à 3:1, par rapport au composé de la formule générale (II).

4. Procédé selon l'une quelconque des revendications 1 à 3, dans lequel, lors de la première étape, du diméthylsulfoxyde, du diméthylacétamide, du diméthylformamide ou de la N-méthyl-2-pyrrolidone est utilisé en tant que solvant.

5. Procédé selon l'une quelconque des revendications 1 à 4, dans lequel, lors de la deuxième étape, H₂O₂ est utilisé pour l'oxydation sélective.

6. Procédé selon l'une quelconque des revendications 1 à 5, dans lequel, lors de la deuxième étape, de l'acide acétique, de l'acide propionique ou du dichlorométhane est utilisé en tant que solvant.

7. Procédé selon l'une quelconque des revendications 1 à 6, dans lequel Na₂WO₄ est utilisé en tant que catalyseur d'oxydation en une quantité de 5 à 15 pour cent en moles, et du peroxyde d'hydrogène est utilisé en une quantité de 3 à 8 équivalents molaires, à chaque fois par rapport au composé de la formule générale (VII).

8. Procédé selon l'une quelconque des revendications 1 à 7, dans lequel, lors de la troisième étape, du bromure d'éthylmagnésium, du chlorure de butylmagnésium ou du chlorure d'iso-propylmagnésium est utilisé en tant que composé organométallique.

9. Procédé selon l'une quelconque des revendications 1 à 8, dans lequel, lors de la troisième étape, du toluène, du tétrahydrofurane ou du 2-méthyltétrahydrofurane est utilisé en tant que solvant.

10. Composés de la formule (III) : 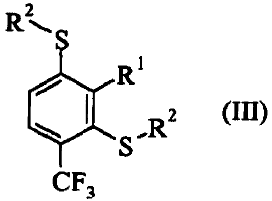 dans laquelle
R¹ et R² signifient indépendamment l'un de l'autre alkyle en C₁-C₄, ou phényle substitué par s radicaux du groupe constitué par chlore, fluor, méthoxy et éthoxy,
s signifie 1, 2 ou 3.

11. Composés selon la revendication 10, dans lesquels R¹ et R² signifient méthyle, éthyle, n-propyle ou iso-propyle ou n-butyle.

12. Composés de la formule (V) : 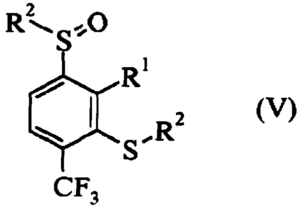 dans laquelle
R¹ et R² signifient indépendamment l'un de l'autre alkyle en C₁-C₄, ou phényle substitué par s radicaux du groupe constitué par chlore, fluor, méthoxy et éthoxy,
s signifie 1, 2 ou 3.

13. Composés selon la revendication 12, dans lesquels R¹ et R² signifient indépendamment l'un de l'autre méthyle, éthyle, n-propyle, iso-propyle ou n-butyle.

14. Composés de la formule (VIII) : 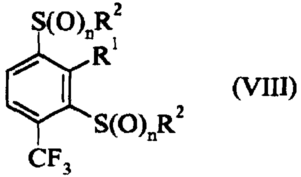 dans laquelle
R¹ et R² signifient indépendamment l'un de l'autre alkyle en C₁-C₄, ou phényle substitué par s radicaux du groupe constitué par chlore, fluor, méthoxy et éthoxy,
s signifie 1, 2 ou 3,
n signifie 1 ou 2.

15. Composés selon la revendication 14, dans lesquels R¹ et R² signifient indépendamment l'un de l'autre méthyle, éthyle, n-propyle, iso-propyle ou n-butyle.
